# EUROPÄISCHE PATENTSCHRIFT

(11) **EP 0 797 098 B1**
(45) Veröffentlichungstag und Bekanntmachung des Hinweises auf die Patenterteilung: **02.10.2002**
(21) Anmeldenummer: 97104679.2
(22) Anmeldetag: 19.03.1997
(51) Int. Cl.: G01N 33/96, G01N 33/72

(54) **Stabilisierung von Bilirubin in Kontrollseren und Kalibratoren**
Method for stabilizing bilirubin in control sera and calibrators
Procédé pour stabiliser la bilirubine dans des sérums de contrôle et des calibrateurs

(30) Priorität: 22.03.1996 DE 19611458; 15.06.1996 DE 19623955
(43) Veröffentlichungstag der Anmeldung: 24.09.1997
(73) Patentinhaber: Roche Diagnostics GmbH, 68298 Mannheim (DE)
(72) Erfinder: Burns, Geoffrey, Dr., Marple, 0999 SK6 7DU (GB); Junius-Comer, Martina, Dr., 82327 Tutzing (GB)

(56) Entgegenhaltungen:
- EP-A- 0 098 562
- FR-A- 2 445 529
- US-A- 4 344 864

## Beschreibung

Gegenstand der Erfindung ist ein stabiles, Bilirubin enthaltendes Kontroll- beziehungsweise Eichserum.

Zur analytischen Überprüfung der methodischen Richtigkeit einzelner Parameter in Humanseren von Patienten werden Kontrollflüssigkeiten serum-ähnlicher Zusammensetzung eingesetzt, die die zu bestimmenden Parameter in bekannten, genau festgelegten Konzentrationen enthalten. Man unterscheidet Spezialkontroll- beziehungsweise Eichseren, die zur Kontrolle beziehungsweise zur Eichung von ganz bestimmten Parametern dienen und sogenannte Universalkontroll- beziehungsweise Eichseren, die zur Kontrolle beziehungsweise zum Eichen möglichst aller gängiger Parameter nach sämtlichen in der Praxis üblicherweise durchgeführten Bestimmungsmethoden einsetzbar sind.

Die Anforderungen, die an solche Produkte im klinisch-chemischen Routinebetrieb gestellt werden, umfassen:
- eine genau bekannte Konzentration der Meßparameter muß eingehalten werden;
- die Konzentrationen müssen im medizinisch relevanten Meßbereich (normal beziehungsweise pathologisch) liegen;
- die Handhabung der Kontroll- beziehungsweise Eichseren muß einfach sein;
- die Kontroll- beziehungsweise Eichseren müssen eine möglichst lange Haltbarkeit besitzen.

Bei einem Einsatz des Kontroll- beziehungsweise Eichserums bei Analyseautomaten werden hohe Anforderungen, besonders an die beiden letzten Punkte (Handhabung und Haltbarkeit) gestellt. Ein in Bezug aufHaltbarkeit kritischer aber wichtiger klinisch-chemischer Parameter ist die gegen Licht und Oxidationsmittel sehr empfindliche Substanz Bilirubin. Bilirubin wird bei der Diagnose, Differenzialdiagnose und Verlaufsbeurteilung des Ikterus gemessen. Während in frisch präparierten Seren humanen oder tierischen Ursprungs oder in Lyophilisaten die Bilirubinkonzentration - Lichteinwirkung ausgeschlossen - selbst bei Temperaturen um 25 °C relativ lange konstant bleibt, unterliegt diese Substanz in Serumpools oder bei länger aufbewahrten Seren auch bei Temperaturen um 4 °C einem raschen Abbau und damit einer Konzentrationsveränderung. Bei Verwendung von Eichseren mit verringerter meßbarer Bilirubinkonzentration würde dies zu falschen Bilirubinwerten in den Serumproben von Patienten und damit zu veränderten Therapieschlüssen führen.

Durch Zusatz von Sulfhydryl-Komponenten, zum Beispiel Dithiothreitol oder Cystein, kann die Bilirubinoxidation verlangsamt werden (Trivin F., Odievre M., Lemonnier A. (1977) Clin. Chem. 23, 541 - 545). In US 4,201,694 wird die Stabilisierung von konjugiertem Bilirubin durch Zusatz einer Sulfhydryl-Komponente bei einem pH von 8.2 - 9.2 im Blutserum beschrieben. Auch Reduktionsmittel wie zum Beispiel Ascorbinsäure (Watson D. (1962) Clin. Sci. 22, 435 - 445) wirken dem oxidativen Bilirubinabbau entgegen.

In WO 92/07088 wird die Stabilisierung von Bilirubin durch die Verwendung eines sauerstofflabilen Reagenzes, Glucose, Glucoseoxidase, eines Wasserstoffdonors, steriler Membranfragmente von Bakterien und einem reagenz-bindenden Agens erreicht. Dieses Verfahren ist jedoch offensichtlich zu aufwendig, zu komplex und zu teuer, um eine industrielle Verwertbarkeit zu gewährleisten. Auch die Stabilisierung von Bilirubin durch Polyole in hohen Konzentrationen, zum Beispiel Ethylenglycol wird beschrieben (Louderback A., Jendrzejezak B., Doumas B.T., Foley Th., (1980) Fresenius Z. Anal. Chem. 301, 145). Die Kontroll- beziehungsweise Eichseren können durch die Zugabe derart hoher Konzentrationen an Polyolen viskos werden. Dies kann bei den Pump- und Pipettierschritten der automatischen Analyse zu Schwierigkeiten und Ungenauigkeiten führen, wodurch die Zugabe hoher Konzentrationen an Polyolen nicht zweckmäßig ist. Bekannt ist auch, daß Metallionen den oxidativen Abbauprozeß des Bilirubins beschleunigen, während gleichzeitige Anwesenheit von metallionenkomplexierenden Reagenzien, wie zum Beispiel EDTA, die Autooxidation verzögern (Fog J., Bakken A. F. (1967) Scand. J. Clin. Lab. Invest. 20, 70 - 72; Fog J., Bugge-Asperheim (1964) Nature 203, 756 - 757; De Ewenson I. W., Gianturco F. A., Gramaccioni P. (1966) Experimentia XXII, 14 - 15; US 4,344,864).

Der Hauptnachteil aller erwähnter Methoden ist die Notwendigkeit, hohe Konzentrationen der vorgenannten Reagenzien einzusetzen. So kann sich ein Stabilisator für einen bestimmten Parameter oftmals bei der Bestimmung anderer Parameter störend auswirken. Reduzierenden SH-Verbindungen können zum Beispiel bei der anschließenden enzymatischen Umsetzung stören. Besonders kritisch ist die negative Wirkung auf PAP-Tests, zum Beispiel Creatinin-PAP oder Harnsäure-PAP. Der PAP-Test beinhaltet eine Indikatorreaktion, bei der H₂O₂ mit Phenol-Aminophenazon (PAP) unter Ausbildung eines roten Farbstoffes reagiert, dessen Absorption proportional der Creatinin-/Harnsäurekonzentration ist.

Bei der Herstellung von Universalkontroll- beziehungsweise Eichseren ist es daher vorteilhaft, so wenig wie möglich Zusatzstoffe zu verwenden. Aufgabe der Erfindung war es, ein Kontrollund Eichserum bereitzustellen, das Bilirubin in einer stabilisierten Form enthält, dessen Konzentration im medizinisch relevanten Meßbereich liegt, dessen Handhabung einfach ist und das eine lange Haltbarkeit besitzt. Die Aufgabe wird durch die in den Ansprüchen näher charakterisierte Erfindung gelöst, die im wesentlichen in der Stabilisierung des Bilirubins in Kontroll- und Eichseren besteht. Dazu gehören die in den Ansprüchen näher charakterisierten chemischen Verbindungen, deren Verwendung, Verfahren zur Herstellung sowie Methoden zum Nachweis von Bilirubin, die Bilirubin schützen und die andere Tests, vor allem den PAP-Test, nicht stören.

Gegenstand der Erfindung ist ein bilirubin-haltiges Kontroll- beziehungsweise Eichserum, das eine chemische Verbindung der allgemeinen Formel (I): 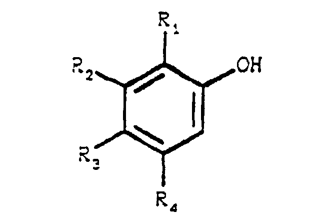 wobei
R1 = H, OH, Alkylrest mit 1 - 8 C-Atomen, COOR5 oder SO₃R5
R2 = H, OH, Alkylrest mit 1 - 8 C-Atomen, COOR5 oder SO₃R5
R3 = H oder OH
R4 = H, COOR5, COR5 oder SO₃R5
R5 = H oder ein Alkylrest mit 1 bis 6 C-Atomen
bedeutet,
und/oder eine chemische Verbindung der allgemeinen Formel (II) 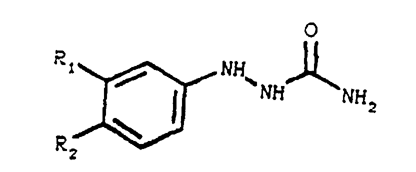 wobei
R1 = H, Halogen: Cl, Br, F oder Alkyl: 1 - 6 C-Atome
R2 = H, Alkyl: C1 - C6 oder Alkoxy: 1 - 6 C-Atome
bedeutet, enthält.

Besonders bevorzugt sind zum Beispiel die Verbindungen der Formel (I) mit:
R1 = OH; R2 = OH; R3 = H; R4 = COOC₃H₇ (=Progallin®) und
R1 = OH; R2 = H; R3 = OH; R4 = COC₄H₉ (= 2,4,5-Trihydroxybutyrophenon)
und die Verbindung der Formel (II) mit:
R1 = H; R2 = CH₃ (= 1-(4-Methylphenyl)-semicarbazid)

Die Verbindungen der allgemeinen Formel (I) beziehungsweise (II) werden in dem Kontrollbeziehungsweise Eichserum in einer Konzentration von 0,001 bis 1,5 mmol/l, bevorzugt von 0,001 bis 0,1 mmol/l (Progallin®) beziehungsweise 0,01 bis 1,5 mmol/l (1-(4-Methoxyphenyl)-semicarbazid, 2,4,5-Trihydroxybutyrophenon, 1-(4-Methylphenyl)-semicarbazid, 1-Phenylsemicarbazid und 1-(3-Chlorphenyl)-semicarbazid) eingesetzt.
Besonders bevorzugt werden die Verbindungen der allgemeinen Formel (I) beziehungsweise (II) in einer Konzentration von 0,03 bis 0,05 mmol/l (Progallin®), 0,03 bis 0,25 mmol/l (1-(4-Methoxyphenyl)-semicarbazid) 0,05 bis 0,25 mmol/l (1-(4-Methylphenyl)-semicarbazid), 0,15 bis 0,25 mmol/l (2,4,5-Trihydroxybutyrophenon und 1-Phenylsemicarbazid) beziehungsweise 0,15 bis 0,3 mmol/l (1-(3-Chlorphenyl)-semicarbazid) eingesetzt.

Der stabilisierende Effekt auf das im Kontroll- beziehungsweise Eichserum enthaltene Bilirubin tritt erst auf, wenn der Stabilisator und das Bilirubin enthaltende Kontroll- beziehungsweise Eichserum gemeinsam in einer Lösung vorliegen. Die Lagerung des Stabilisators und des Kontroll- beziehungsweise Eichserums kann folgendermaßen erfolgen:
Kontroll- beziehungsweise Eichserum und Stabilisator zusammen in lyophilisierter Form oder
Kontroll- beziehungsweise Eichserum und Stabilisator separat in lyophilisierter Form oder
Kontroll- beziehungsweise Eichserum lyophilisiert, Stabilisator in flüssiger Form oder
Kontroll- beziehungsweise Eichserum und Stabilisator zusammen in flüssiger Form.
Um einen zusätzlichen stabilisierenden Effekt zu erreichen, können das Kontrollbeziehungsweise Eichserum und der Stabilisator bei niedrigen Temperaturen, gegebenenfalls unter dem Gefrierpunkt aufbewahrt werden.

Das erfindungsgemäße Kontroll- beziehungsweise Eichserum kann neben den Analytparametern noch zusätzliche Hilfsstoffe, beispielsweise weitere Analytstabilisatoren, Detergenzien und Konservierungsstoffe enthalten. Dabei ist bei dem Zusatz dieser Hilfsstoffe stets darauf zu achten, daß die Messung bestimmter Parameter nicht störend beeinflußt wird.

Durch eine Aufbewahrung des Reagenzes unter einer Schutzgasatmosphäre, besonders einer N₂-Schutzgasatmosphäre, wird eine besonders hohe Stabilität des Bilirubins erzielt. Die Schutzgasatmosphäre wirkt sich außer auf die Stabilität des Bilirubins sehr günstig auf die Stabilität anderer Parameter, wie zum Beispiel Harnsäure aus. Die Stabilisierung des Bicarbonatwertes wird durch Zusatz von ca. 0,3 Vol.-% CO₂ zum N₂-Inertgas erreicht. Neben diesem Analytstabilisator können noch weitere einzelne analytstabilisierende Hilfsstoffe zugesetzt werden. So wirkt sich zum Beispiel ein Zusatz von EDTA stabilisierend auf den Eisenwert aus. Die Zugabe einer verdünnten Fe-Lösung bei der Herstellung des Kontrollbeziehungsweise Eichserums in Gegenwart von EDTA, bevorzugt in einem Verhältnis von 1:1,1 (Fe/EDTA), führt zu stabilisierten Fe-Komplexen, die nicht von Lipoproteinen beziehungsweise Rinderserumalbumin zerstört werden.

Die Zugabe von Creatin wirkt sich positiv auf die Stabilität des Parameters Creatinin aus, da die Creatinin-Hydrolyse hierdurch verhindert oder zumindest stark vermindert wird.

Für den Triglycerid-Parameter wird in bisher gebräuchlichen Kontroll- beziehungsweise Eichseren eine Eigelbfraktion eingesetzt. Trübungen sind bei einer solchen Zusammensetzung nicht auszuschließen. Durch den Ersatz dieser Eigelbfraktion durch Glycerin als Triglycerid kann diese potentielle Störung gänzlich vermieden werden. Glycerin zeigt auch bei längerer Lagerung keine Neigung zur Trübungsbildung und ist daher für den Einsatz im erfindungsgemäßen Kontroll- beziehungsweise Eichserum vorzüglich geeignet.

Der pH-Wert des Kontroll- beziehungsweise Eichserums wird durch Verwendung eines Puffers auf einen pH von vorzugsweise 6.0 bis 8.6 eingestellt.

Das erfindungsgemäße Kontroll- beziehungsweise Eichserum ist im ungeöffneten Zustand bei 4 °C über 12 Monate hinaus stabil. Durch eine Aufbewahrung bei -20 °C kann diese Stabilität noch deutlich erhöht werden. Auch in geöffnetem Zustand bei 10 °C ist das Produkt mehrere Tage haltbar. Es eignet sich hervorragend als Qualitätskontroll- und Eichserum für Parameter der klinischen Diagnostik, insbesondere unter Berücksichtigung von Bilirubin. Besonders bevorzugt wird das erfindungsgemäße Kontroll- beziehungsweise Eichserum bei der automatischen klinischen Diagnostik verwendet.

Ein weiterer Gegenstand ist ein Verfahren zur Herstellung des erfindungsgemäßen Kontrollbeziehungsweise Eichserums, wobei von einer Rinderserumalbuminlösung ausgegangen wird, die mit den gewünschten Parametern bis zur gewünschten Parameterkonzentration vermischt wird, wobei die angegebenen und gegebenenfalls noch andere Hilfsmittel zugesetzt werden.

Ein weiterer Gegenstand ist die Verwendung der Verbindungen der allgemeinen Formeln (I) und/oder (II) zur Stabilisierung von Bilirubin in Lösungen.

Ein weiterer Gegenstand ist eine Methode zur Erhöhung der Lagerstabilität von bilirubin-haltigen Eich- und Kontrollseren, die dadurch erreicht wird, daß Verbindungen der allgemeinen Formeln (I) und/oder (II) zur Stabilisierung von Bilirubin in Lösungen zugegeben werden.

Weiterhin ist Gegenstand der Erfindung eine Methode zum Nachweis von Bilirubin, die gekennzeichnet ist durch Messung des Bilirubingehaltes in der Probe, Messung von Bilirubin in einem erfindungsgemäßen Kontroll- oder Eichserum und anschließend Bestimmung des Bilirubingehaltes der Probe durch Vergleich der beiden Messungen. Die Bilirubin-Bestimmung in der Probe beziehungsweise im Kontroll- oder Eichserum erfolgt nach dem Fachmann bekannten Methoden.

Gegenstand der Erfindung ist auch ein Kit zur Messung von Bilirubin in Proben, das das erfindungsgemäße Kontroll- und Eichserum enthält und in dem zusätzlich die zur Messung notwendigen Reagenzien enthalten sind.

Die Erfindung wird durch folgende Beispiele näher erläutert:

### Beispiel 1

### Stabilisierung von Bilirubin in Kontrollseren

Für die Untersuchung der Bilirubinstabilität wird ein Progallin®-freies PPU (Precipath U: Universal-Kontrollserum mit Sollwerten im pathologischen Bereich; Boehringer Mannheim GmbH, Id.Nr. 171 760) mit den Stabilisatoren aufgestockt. Ein erstes Screening erfolgt mit verschiedenen Konzentrationen an Stabilisatoren. Die Proben werden in der Dunkelheit gelagert und anschließend D- und T-Bilirubinstabilitäten nach 4 Stunden bei 25 °C gemessen (D-Bilirubin, auch δ-Bilirubin oder Bd ist kovalent an Albumin gebunden und hat darum eine Halbwertszeit von ca. 18 Tagen. T-Bilirubin steht für Gesamtbilirubin - konjugiert und nicht konjugiert). Die Messung von D- und T-Bilirubin erfolgte mit den entsprechenden Testkits von Boehringer Mannheim GmbH, Deutschland:
T-Bilirubin: Ident-No. 104 0804, Charge 64430201 (HiCo, DPD-Methode)
D-Bilirubin: Ident-No. 110 9774, Charge 64548901 (Jendrassik-Methode)

Tabelle 1 zeigt die Ergebnisse des Screenings.

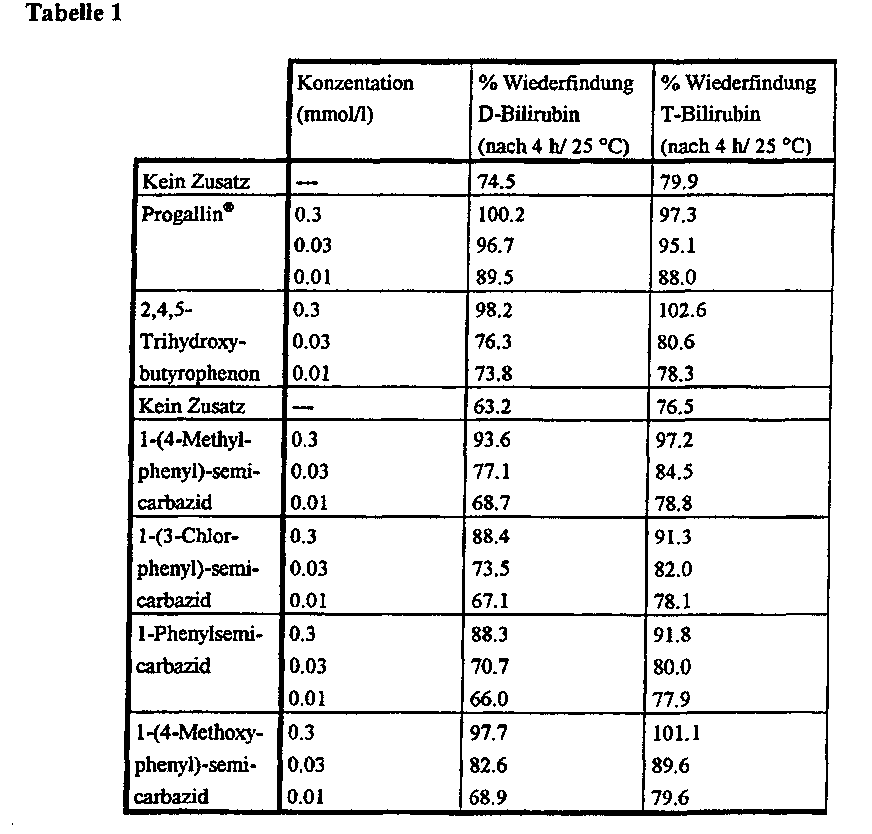

Die beanspruchten Substanzen zeigen einen positiven Einfluß auf die Stabilität von D- und T-Bilirubin.

Tabellen 1 und 2 zeigen die Ergebnisse der Feinoptimierung der Konzentrationen dieser Substanzen. Gezeigt wird die Wiederfindung von D-Bilirubin (nach 4 Stunden bei 25 °C) und Harnsäure gegen die Konzentration der entsprechenden Stabilisatoren. Die erfindungsgemäß eingesetzten Substanzen zeigen eine Stabilisierung von Bilirubin.

### Beispiel 2

### Störungen im Creatinin-PAP- / Harnsäure-PAP-Test

Auch hier wurden verschiedene Konzentrationen der beanspruchten Substanzen gemäß Tabelle 2 eingesetzt.

Die Proben wurden wiederum in der Dunkelheit gelagert. Die Störungen im Harnsäure- und Creatinin-PAP-Test wurden jedoch nur einmal am Anfang des Experimentes gemessen. Für die Bestimmungen von Harnsäure- und Creatinin-PAP wurden die entsprechenden Testkits von Boehringer Mannheim GmbH, Deutschland, verwendet und entsprechend den Angaben des Herstellers die Tests durchgeführt.
Harnsäure-PAP: Ident-No. 144 6765, Charge 64834001 und
Creatinin-PAP: Ident-No. 148 9291, Charge 64720101

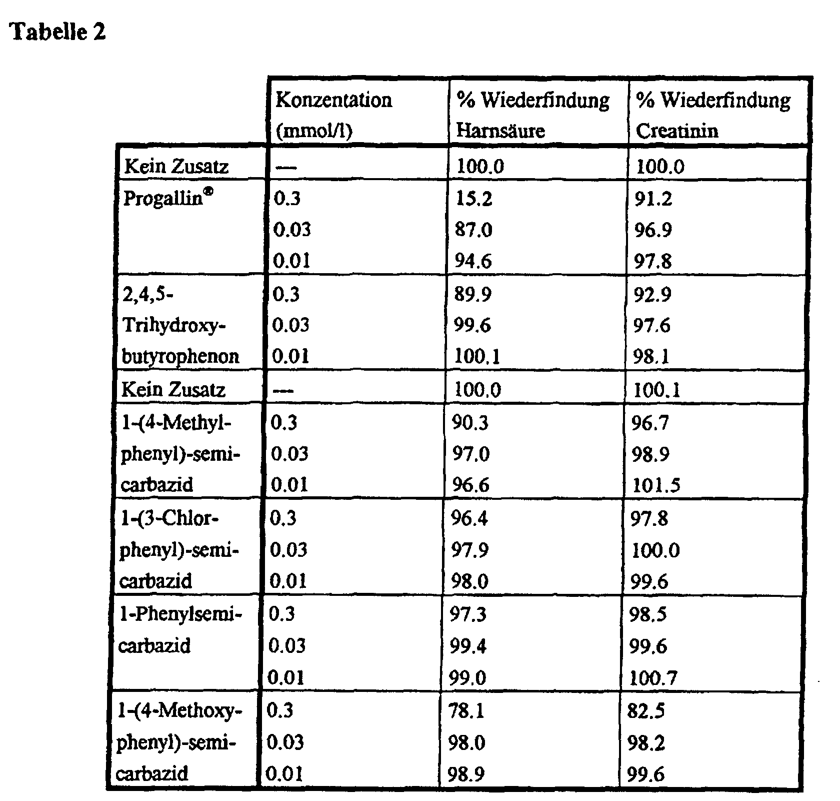

Die beanspruchten Substanzen zeigen unterschiedliche Effekte auf Harnsäure-PAP während die Auswirkungen auf den Creatinin-PAP einheitlich sind. Es ist möglich, mit der Verwendung der dargestellten Ergebnisse eine optimale Konzentration für die Stabilisatoren festzustellen, die eine hohe Stabilität von Bilirubin und eine niedrige Störung der PAP-Teste garantiert:

| | |
|---|---|
| Progallin® | 0,001 - 0,1 mmol/l |
| 2,4,5-Trihydroxybutyrophenon | 0,01 - 1,5 mmol/l |
| 1-(4-Methylphenyl)-semicarbazid | 0,01 - 1,5 mmol/l |
| 1-Phenylsemicarbazid | 0,01 - 1,5 mmol/l |
| 1-(3-Chlorphenyl)-senucarbazid | 0,01 - 1,5 mmol/l |
| 1-(4-Methoxyphenyl)-semicarbazid | 0,01 - 1,5 mmol/l |

besonders bevorzugt sind:

| | |
|---|---|
| Progallin® | 0,03 - 0,05 mmol/l |
| 2,4,5-Trihydroxybutyrophenon | 0,15 - 0,25 mmol/l |
| 1-(4-Methylphenyl)-semicarbazid | 0,05 - 0,25 mmol/l |
| 1-Phenylsemicarbazid | 0,15 - 0,25 mmol/l |
| 1-(3-Chlorphenyl)-semicarbazid | 0,15 - 0,30 mmol/l |
| 1-(4-Methoxyphenyl)-semicarbazid | 0,03 - 0,25 mmol/l |

Es kann folglich gezeigt werden, daß die beanspruchten Substanzen bei entsprechender Optimierung sowohl als Bilirubinstabilisatoren als auch als nicht-störende Substanzen im Creatinin-PAP und Harnsäure-PAP eingesetzt werden können.

Für bilirubin-haltige Eichseren oder Kontrollseren, die eine andere Zusammensetzung als das in den Beispielen 1 und 2 verwendete Serum besitzen, können sich bevorzugte Konzentrationsbereiche ergeben, die geringfügig von den hier genannten Bereichen abweichen. Ein Fachmann kann anhand einfacher Untersuchungen, wie in Beispiel 1 und 2 gezeigt, die jeweilig bevorzugten Konzentrationen leicht ermitteln.

## Patentansprüche

1. Bilirubin-haltiges Kontroll- beziehungsweise Eichserum, **dadurch gekennzeichnet, daß** eine chemische Verbindung der allgemeinen Formel (I): 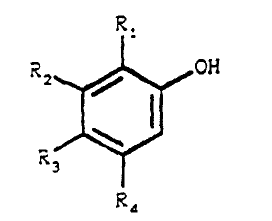 wobei
R1 = H, OH-Alkylrest mit 1 - 8 C-Atomen, COOR5 oder SO₃R5
R2 = H, OH, Alkylrest mit 1 - 8 C-Atomen, COOR5 oder SO₃R5
R3 = H oder OH
R4 = H, COOR5, COR5 oder SO₃R5
R5 = H oder Alkylrest mit 1 - 6 C-Atomen
bedeutet
und/oder eine chemische Verbindungen der allgemeinen Formel (II): 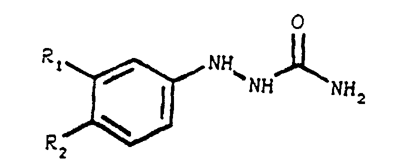 wobei
R1 = H, Halogen oder Alkylrest mit 1 - 6 C-Atomen
R2 = H, Alkylrest mit 1 - 6 C-Atomen, Alkoxyrest mit 1 - 6 C-Atomen,
bedeutet,
enthalten ist.

2. Kontroll- beziehungsweise Eichserum gemäß Anspruch 1, **dadurch gekennzeichnet, daß** die chemische Verbindung der allgemeinen Formel (I)
R1 = OH, R2 = OH, R3 = H, R4 = COOC₃H₇
oder
R1 = OH, R2 = H, R3 = OH, R4 = COC₄H₉
ist.

3. Ein Kontroll- beziehungsweise Eichserum gemäß Anspruch 1 und 2, **dadurch gekennzeichnet, daß** die chemische Verbindung der allgemeinen Formel (II)
R1 = H, R2 = CH₃
ist.

4. Ein Kontroll- beziehungsweise Eichserum gemäß einem der Ansprüche 1 bis 3, das zusätzliche Konservierungsmittel oder/und Analytstabilisatoren enthält.

5. Ein Kontroll- beziehungsweise Eichserum gemäß Anspruch 1, das unter Schutzgas aufbewahrt wird.

6. Ein Kontroll- beziehungsweise Eichserum gemäß einem der Ansprüche 1 - 5, bei dem die Einsatzkonzentrationen der chemischen Verbindungen der Formeln (I) und/oder (II) zwischen 0,001 und 1,5 mmol/l liegen.

7. Verwendung chemischer Verbindungen der Formeln (I) und/oder (II) zur Stabilisierung von Bilirubin in Lösungen.

8. Verfahren zur Stabilisierung von Bilirubin in einer Lösung, **dadurch gekennzeichnet, daß** eine Verbindung der Formel (I) und/oder (II) der bilirubin-haltigen Lösung zugegeben wird.

9. Verfahren zur Herstellung eines Kontroll- beziehungsweise Eichserums **dadurch gekennzeichnet, daß** eine Verbindung der Formel (I) und/oder eine Verbindung der Formel (II) einer bilirubin-haltigen Kontroll- bzw. Eichserum zugesetzt wird.

10. Eine Methode zum Nachweis von Bilirubin durch
a) Messung von Bilirubin in der Probe
b) Messung von Bilirubin in einem Kontroll- oder Eichserum mit einem definierten Bilirubingehalt
c) Bestimmung des Bilirubingehaltes durch Vergleich der Messung a) mit der Messung b), **dadurch gekennzeichnet, daß** ein Kontroll- oder Eichserum gemäß einem der Ansprüche 1 bis 6 verwendet wird.

11. Eine Methode zur Erhöhung der Lagerstabilität von bilirubin-haltigen Eichbeziehungsweise Kontrollseren, **dadurch gekennzeichnet, daß** eine chemische Verbindung der Formel (I) und/oder (II) gemäß Anspruch 1 zugegeben wird.

12. Kit zur Messung von Bilirubin in Proben, **dadurch gekennzeichnet, daß** ein bilirubin-haltiges Kontroll- beziehungsweise Eichserum gemäß einem der Ansprüche 1 bis 6 und zusätzliche zur Messung erforderliche Reagenzien enthalten sind.

## Claims

1. Control or calibration serum containing bilirubin, **characterized in that** it contains a chemical compound of the general formula (I): 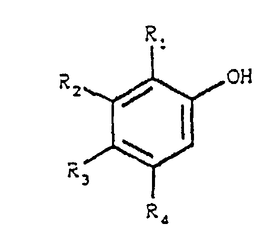
in which R1 = H, OH, an alkyl residue with 1-8 C atoms, COOR5 or SO₃R5
R2 = H, OH, an alkyl residue with 1-8 C atoms, COOR5 or SO₃R5
R3 = H or OH
R4 = H, COOR5, COR5 or SO₃R5
R5 = H or an alkyl residue with 1 - 6 C atoms
and/or a chemical compound of the general formula (II): 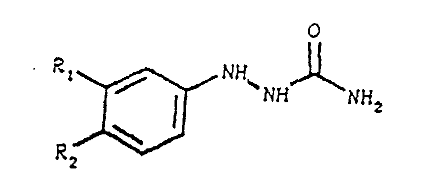
in which R1 = H, halogen or an alkyl residue with 1 - 6 C atoms
R2 = H, an alkyl residue with 1 - 6 C atoms, an alkoxy residue with 1 - 6 C atoms.

2. Control or calibration serum as claimed in claim 1, **characterized in that** in the chemical compound of the general formula (I)
R1 = OH, R2 = OH, R3 = H, R4 = COOC₃H₇
or
R1 = OH, R2 = H, R3 = OH, R4 = COC₄H₉.

3. Control or calibration serum as claimed in claim 1 and 2, **characterized in that** in the chemical compound of the general formula (II)
R1 = H and R2 = CH₃.

4. Control or calibration serum as claimed in one of the claims 1 to 3, **characterized in that** it additionally contains preservatives or/and analyte stabilizers.

5. Control or calibration serum as claimed in claim 1, which is stored under a protective gas.

6. Control or calibration serum as claimed in one of the claims 1 - 5, in which the chemical compounds of formulae (I) and/or (II) are used at a concentration between 0.001 and 1.5 mmol/l.

7. Use of chemical compounds of formulae (I) and/or (II) to stabilize bilirubin in solutions.

8. Method for stabilizing bilirubin in a solution, **characterized in that** a compound of formula (I) and/or (II) is added to the solution containing bilirubin.

9. Process for the production of a control or calibration serum, **characterized in that** a compound of formula (I) and/or a compound of formula (II) is added to a control or calibration serum containing bilirubin.

10. Method for detecting bilirubin by
a) measuring bilirubin in the sample
b) measuring bilirubin in a control or calibration serum containing a defined amount of bilirubin
c) determining the bilirubin content by comparing measurement a) with measurement b), **characterized in that** a control or calibration serum as claimed in one of the claims 1 to 6 is used.

11. Method for increasing the storage stability of calibration or control sera containing bilirubin, **characterized in that** a chemical compound of formula (I) and/or (II) as claimed in claim 1 is added.

12. Kit for measuring bilirubin in samples, **characterized in that** it contains a control or calibration serum containing bilirubin as claimed in one of the claims 1 to 6 and additional reagents required for the measurement.

## Revendications

1. Sérum étalon ou témoin contenant de la bilirubine, **caractérisé en ce qu'**un composé chimique de formule générale (I) : 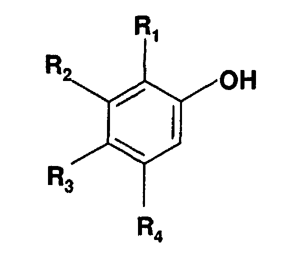 dans laquelle
R1 = H, OH, résidu alkyle ayant 1 à 8 atomes de C, COOR5 ou SO₃R5
R2 = H, OH, résidu alkyle ayant 1 à 8 atomes de C, COOR5 ou SO₃R5
R3 = H ou OH
R4 = H, COOR5, COR5 ou SO₃R5
R5 = H ou un résidu alkyle ayant 1 à 6 atomes de C
et/ou un composé chimique de formule générale (II) : 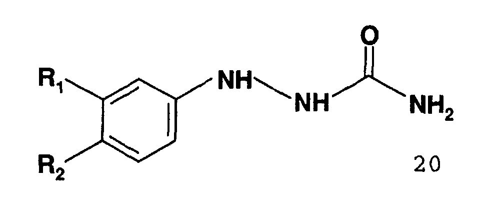 dans laquelle
R1 = H, un atome d'halogène ou un résidu d'alkyle ayant 1 à 6 atomes de C
R2 = H, un résidu alkyle ayant 1 à 6 atomes de C, un résidu alcoxy ayant 1 à 6 atomes de C,
y sont contenus.

2. Sérum étalon ou témoin selon la revendication 1, **caractérisé en ce que** le composé chimique de formule générale (I) est celui où
R1 = OH, R2 = OH, R3 = H, R4 = COOC₃H₇
ou
R1 = OH, R2 = H, R3 = OH, R4 = COC₄H₉.

3. Sérum étalon ou témoin selon la revendication 1 et 2, **caractérisé en ce que** le composé chimique de formule générale (II) est celui où
R1 = H, R2 = CH₃.

4. Sérum étalon ou témoin selon l'une quelconque des revendications 1 à 3, qui contient des conservateurs et/ou des stabilisants d'analyte supplémentaires.

5. Sérum étalon ou témoin selon la revendication 1, qui est conservé sous un gaz protecteur.

6. Sérum étalon ou témoin selon l'une quelconque des revendications 1 à 5, dans lequel les concentrations mises en oeuvre des composés chimiques des formules (I) et/ou (II) sont comprises entre 0,001 et 1,5 mmole/l.

7. Utilisation de composés chimiques de formules (I) et/ou (II) pour stabiliser la bilirubine dans des solutions.

8. Procédé pour stabiliser la bilirubine dans une solution, **caractérisé en ce que** l'on ajoute un composé de formule (I) et/ou (II) à la solution contenant la bilirubine.

9. Procédé de préparation d'un sérum étalon ou témoin **caractérisé en ce que** l'on ajoute à un sérum étalon ou témoin contenant la bilirubine, un composé de formule I et/ou un composé de formule (II).

10. Procédé de détection de la bilirubine par
a) mesure de la bilirubine dans l'échantillon,
b) mesure de la bilirubine dans un sérum étalon ou témoin ayant une teneur définie en bilirubine,
c) détermination de la teneur en bilirubine en comparant la mesure a) à la mesure b), **caractérisé en ce qu'**un sérum étalon ou témoin selon l'une quelconque des revendications 1 à 6 est employé.

11. Procédé pour augmenter la stabilité à l'entreposage de sérums étalons ou témoins, contenant de la bilirubine, **caractérisé en ce que** l'on y ajoute un composé chimique de formule (I) et/ou (II) selon la revendication 1.

12. Trousse pour mesurer la bilirubine dans des échantillons, **caractérisé en ce qu'**un sérum étalon ou témoin contenant de la bilirubine selon l'une quelconque des revendications 1 à 6 et des réactifs supplémentaires nécessaires à la mesure y sont contenus.
